(19) Europäisches Patentamt
European Patent Office
Office européen des brevets

(11) Veröffentlichungsnummer: **0 001 276**
**B1**

(12) # EUROPÄISCHE PATENTSCHRIFT

(21) Anmeldenummer: **78100917.0**

(51) Int. Cl.$^3$: **C 07 C 143/70**

(22) Anmeldetag: **18.09.78**

(54) **Verfahren zur Herstellung von Sulfonsäurechloriden**

(30) Priorität: **28.09.77 DE 2743542**

(43) Veröffentlichungstag der Anmeldung:
**04.04.79 Patentblatt 79/07**

(45) Bekanntmachung des Hinweises auf die Patenterteilung:
**07.01.81 Patentblatt 81/01**

(84) Benannte Vertragsstaaten:
**BE CH DE FR GB LU NL**

(56) Entgegenhaltungen:
**BE - A - 553 871**
**DE - A - 1 963 383**
**DE - A - 2 240 883**
**DE - B - 1 593 906**

**HOUBEN-WEYL "Methoden der organischen Chemie"**
**4. Auflage, Band IX, 1955,**
**Thieme Verlag Stuttgart**
**Seiten 567—568**

(73) Patentinhaber: **BAYER AG**
**Zentralbereich Patente, Marken und Lizenzen**
**D - 5090 Leverkusen 1,**
**Bayerwerk (DE)**

(72) Erfinder: **Blank, Heinz-Ulrich, Dr.**
**Am Geusfelde 35**
**D - 5068 Odenthal (DE)**
**Pfister, Theodor, Dr.**
**Willer Strasse 51**
**D - 5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 001 276 B1**

Courier Press, Leamington Spa, England.

Verfahren zur Herstellung von Sulfonsäurechloriden

Die Erfindung betrifft ein Verfahren zur Herstellung von Sulfonsäurechloriden.

Es ist bekannt (Houben-Weyl, Methoden der organischen Chemie, Band 9, Seite 567 (1955), daß bei der Einwirkung von Phosgen auf aromatische Sulfonsäuren oder deren Alkalisalze in einem indifferenten Lösungsmittel bei 140—180°C die entsprechenden Sulfochloride gebildet werden.

Ebenso ist bekannt, daß aromatische Sulfonsäurechloride aus den entsprechenden Sulfonsäuren und Phosgen in Gegenwart von Dimethylformamid und tertiären Aminen bei Temperaturen unter 100°C hergestellt werden können (DT—OS 1 963 383).

In der DT—AS 1 593 906 wird die Herstellung von aromatischen Sulfonsäurechloriden aus Sulfonsäuren und Phosgen in Gegenwart von tertiären organischen Säureamiden als Katalysatoren beschrieben. Bei diesen Verfahren wird Phosgen stets im Überschuß gegenüber den Sulfonsäuren eingesetzt.

Es wurde ein Verfahren zur Herstellung von Sulfonsäurechloriden der Formel

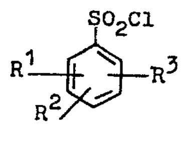

(I)

worin

$R^1$, $R^2$ und $R^3$ gleich oder verschieden sind und Wasserstoff, einen niederen Alkyl- oder einen Cycloalkylrest, Halogen, Nitro, Aryl, Aralkyl, Arylether oder einen Rest

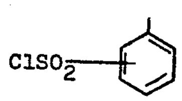 , 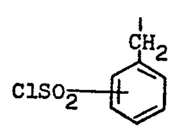 oder 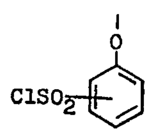 bedeuten,

wobei die Reste $R^1$, $R^2$ und $R^3$ durch Halogen, Nitro, Niederalkyl, Aryl, Aroxy, Alkoxy oder Aralkyl substituiert sein können, oder wo benachbarte Reste

$R^1$ und $R^2$ zu einem gegebenenfalls durch eine Sulfonsäure-chloridgruppe substituierten cycloaliphatischen oder aromatischen carbocyclischen Ring verknüpft sind, aus Sulfonsäurender Formel

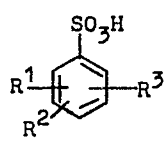

(II)

worin

$R^1$, $R^2$ und $R^3$ die oben genannte Bedeutung haben, und Phosgen in Gegenwart von Katalysatoren aus der Klasse der N,N-Dialkylcarbonsäureamide gefunden, bei dem man die Umsetzung in Gegenwart eines Sulfonierungsmittels durchführt.

Niedere Alkylreste ($R^1$ bis $R^3$) können geradkettige oder verzweigte Alkylreste mit 1 bis 6, bevorzugt 1 bis 4, Kohlenstoffatomen sein. Beispielweise seien genannt: Methyl, Athyl, Propyl, Iso-propyl, Butyl, Iso-butyl, Pentyl, Iso-pentyl, Hexyl und Iso-hexyl.

Als Cycloalkylreste ($R^1$ bis $R^3$) seien beispielsweise Cyclopentyl und Cyclohexyl, bevorzugt Cyclohexyl, genannt.

Als Halogene ($R^1$ bis $R^3$) seien Fluor, Chlor, Brom und Jod, bevorzugt Fluor, Chlor und Brom, genannt.

Als Arylreste ($R^1$ bis $R^3$) seien beispielsweise Phenyl und Naphthyl, bevorzugt Phenyl, genannt.

Als Aralkylreste ($R^1$ bis $R^3$) kommen beispielsweise solche mit 6 bis 18 Kohlenstoffatomen, deren aliphatischer Teil 1 bis 6 Kohlenstoffatome enthält und deren aromatischer Teil einen Rest aus der Benzolreihe darstellt, in Frage. Beispielsweise seien die folgenden araliphatischen Reste genannt:

Benzyl, $\beta$-Phenyl-äthyl, $\delta$-Phenyl-propyl und $\beta$-Phenyl-n-hexyl, bevorzugt Benzyl.

Als Arylätherrest ($R^1$ bis $R^3$) sei insbesondere der Phenoxy-rest genannt.

Durch Verknüpfung der benachbarten Reste $R^1$ und $R^2$ zu einem cycloaliphatischen oder aromatischen Ring entstehen kondensierte Ringsysteme, wie Indan, Tetralin, Inden und Naphthalin, bevorzugt Naphthalin.

Es ist selbstverständlich möglich, daß die Reste $R^1$ bis $R^3$ durch weitere Reste, die unter den Bedingungen des erfindungsgemäßen Verfahrens nicht verändert werden, substituiert sind. Beispielsweise sei Halogen, Nitro, Niederalkyl, Aryl, Aroxy, Alkoxy und Aralkyl genannt.

Als bevorzugte Sulfonsäuren seien Verbindungen der Formel

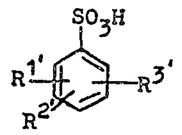

(III)

worin

$R^{1'}$, $R^{2'}$ und $R^{3'}$ gleich oder verschieden sind und Wasserstoff, einen Alkylrest mit 1 bis 4 Kohlenstoffatomen, Fluor, Chlor, Brom, Nitro, Phenyl, Phenoxy oder Benzyl bedeuten

oder wo benachbarte Reste

$R^{1'}$ und $R^{2'}$ zu einem cycloaliphatischen oder aromatischen carbocyclischen Ring mit 6 Ringgliedern verknüpft sind,

gennant.

Beispielsweise seien die folgenden Sulfonsäuren genannt: Benzolsulfonsäure, 2-, 3- und 4-Toluolsulfonsäure, 2-, 3- und 4-Chlor-benzolsulfonsäure, 2.5- und 3.4-Dichlorbenzolsulfonsäure, 3-Nitro-benzosulfonsäure, 4-Chlor-3-nitro-benzosulfonsäure, 6-Chlor-3-nitro-benzosulfonsäure, 2-Chlor-toluol-4-sulfonsäure, 4-Chlor-toluol-2-sulfonsäure, 2-Nitro-toluol-4-sulfonsäure, 4-Nitro-toluol-2-sulfonsäure, 1- und 2-Naphthalinsulfonsäure, 5-Nitro-naphthalin-1-sulfonsäure, 5-Nitro-naphthalin-2-sulfonsäure, 4-Biphenylsulfonsäure, 4.4-Biphenyldisulfonsäure, 4-Phenoxy-benzolsulfonsäure und Diphenylmethan-4-sulfonsäure.

Insbesondere bevorzugtes Ausgangsprodukt ist Benzosulfonsäure.

Als Sulfonierungsmittel seien Schwefelsäure, Schwefeltrioxid, Chlorsulfonsäure und Sulfurylchlorid, bevorzugt Schwefelsäure, Chlorsulfonsäure und Sulfurylchlorid genannt. Auch Gemische dieser Sulfonierungsmittel, z.B. Oleum, können eingesetzt werden.

Im allgemeinen können im erfindungsgemäßen Verfahren bis zu ungefähr 30 Gew.-%, bevorzugt 2—20 Gew.-% und insbesondere 4—15 Gew.-% Sulfonierungsmittel eingesetzt werden, wobei die Menge des Sulfonierungsmittels auf die eingesetzte Menge Benzolsulfonsäure bezogen ist.

Das Sulfonierungsmittel kann der Sulfonsäure vor deren Umsetzung mit Phosgen beigemischt werden. Es ist aber auch möglich, während der Umsetzung der Sulfonsäure mit Phosgen das Sulfonierungsmittel zuzugeben. Ebenso gut ist es möglich, das Sulfonierungsmittel vor der Zugabe der Sulfonsäure zusammen mit Phosgen und Dimethylformamid vorzulegen. Vorzugsweise wird die berechnete Menge an Sulfonierungsmittel im Gemisch mit der Sulfonsäure eingesetzt.

Für das erfindungsgemäße Verfahren können reine oder technische Sulfonsäuren eingesetzt werden.

Als herstellungsbedingte Verunreinigungen kommen z.B. in Frage: Wasser, Disulfonsäuren, Sulfone, Sulfonsulfonsäuren, Sulfonsäureanhydride und nicht umgesetzte Ausgangsprodukte. Die Sulfonsäuren können außerdem noch Reste von Sulfonierungsmitteln oder von Zusätzen enthalten, die bei ihrer Herstellung zur Verhinderung der Sulfonbildung verwendet wurden.

Vorzugsweise verwendet man Sulfonsäuren, die durch Reaktion aromatischer Verbindungen mit Schwefeltrioxid in bekannter Weise hergestellt worden sind.

Sulfonsäuren, die durch Umsetzung aromatischer Verbindungen mit anderen Sulfonierungsmitteln wie z.B. Schwefelsäure, Oleum oder Chlorsulfonsäure hergestellt worden sind, können in gleicher Weise eingesetzt werden.

Falls die eingesetzten Sulfonsäuren durch die Herstellung geringe Mengen an Sulfonierungsmittel enthalten, wird vorteilhafterweise der Zusatz an Sulfonierungsmittel darauf abgestimmt.

Phosgen wird nach dem erfingungsgemäßen Verfahren im allgemeinem im Überschuß über die stöchiometrisch erforderliche Menge von 1 Mol Phosgen je Mol Sulfonsäure verwendet. Gewöhnlich wird ein Überschuß von bis zu 100% Phosgen eingesetzt. Selbstverständlich können auch größere Phosgenüberschüsse verwendet werden, jedoch bringt dies im allgemeinen keinen Vorteil. Nicht umgesetztes, überschüssiges Phosgen kann zurückgewonnen und wiederverwendet werden.

Das erfindungsgemäße Verfahren wird so durchgeführt, daß das Phosgen während der Umsetzung stets im Überschuß gegenüber der Sulfonsäure im Reaktionsgemisch vorhanden ist.

Das erfindungsgemäße Verfahren wird unter Verwendung von Katalysatoren aus der Klasse der N,N-Dialkylcarbonsäureamide durchgeführt. Als solche kommen bekannte Verbindungen in Frage, wie sie beispielsweise in US—PS 3 673 247, in FR—PS 1 513 906 und in DT—OS 1 593 906, 1 963 383 und 2 240 883 beschrieben sind.

Im allgemeinen werden N,N-Dialkylcarbonsäureamide, wie z.B. Dimethylformamid oder Dimethylacetamid, vorzugsweise Dimethylformamid, bevorzugt verwendet.

Die Katalysatoren werden im allgemeinen in Mengen von 1 bis 15%, bevorzugt von 2 bis 10%, bezogen auf das Gewicht der Sulfonsäuren, eingesetzt.

Für das erfindungsgemäße Verfahren sind Lösungsmittel nicht erforderlich. Es kann jedoch auch unter Verwendung von Lösungs- oder Verdünnungsmitteln, welche gegen die Reaktions-partner inert sind, gearbeitet werden. Als solche kommen hauptsächlich Halogenkohlenwasserstoffe, wie Di-, Tri- oder Tetrachlormethan, Di-, Tri-, Tetra- und Pentachloräthan sowie Tri- und Tetra-Chloräthylen, Aromaten, wie z.B. Benzol, Toluol, insbesondere Chlorbenzol, o-, m- und p-Dichlorbenzol sowie anorganische Lösungsmittel, wie beispielsweise flüssiges Schwefeldioxid und überschüssiges Phosgen in

Betracht. Auch das Endprodukt des Verfahrens, das Sulfochlorid, kann gegebenenfalls als Lösungsmittel verwendet werden.

Das erfindungsgemäße Verfahren kann im Temperaturbereich von etwa 0°C bis 100°C durchgeführt werden. Bevorzugt wird der Bereich zwischen 20 und 80°C, insbesondere der Bereich zwischen 40 und 70°C.

Der Druck ist nicht wesentlich für die Durchführung des erfindungsgemäßen Verfahrens. Mann kann es bei Normaldruck, erhöhtem oder vermindertem Druck durchführen. Im allgemeinen wird es bei Atmosphärendruck durchgeführt. Bei Verwendung von niedrig siedenden Lösungsmitteln oder einem großen Überschuß an Phosgen arbeitet man bei Rückflußbedingungen und einem der erforderlichen Sumpftemperatur angepaßten Druck.

Das erfindungsgemäße Verfahren kann sowohl diskontinuierlich als auch kontinuierlich durchgeführt werden.

Bei der diskontinuierlichen Durchführung in einem Rührkessel werden vorteilhaft der Katalysator und mindestens eine diesem äquivalente Menge Phosgen vorgelegt und die Sulfonsäure und das Phosgen dann so eindosiert, daß letzteres im Gemisch immer im Überschuß vorhanden ist. Die Zugabe der Sulfonsäure erfolgt zweckmäßig mit etwa der gleichen Geschwindigkeit mit der das Sulfochlorid gebildet wird.

Die Bildung des Sulfochlorids kann durch die Bestimmung der Nebenprodukte Kohlendioxid und Chlorwasserstoff mit bekannten analytischen Methoden verfolgt werden.

Das erfindungsgemäße Verfahren kann auch in einer Kaskade von Rührkesseln kontinuierlich durchgeführt werden. Das Phosgen, welches auch hierbei immer im Überschuß gegenüber der Sulfonsäuren vorhanden sein muß, kann bei dieser Verfahrenweise im Kreis geführt werden.

Zu dem kontinuierlichen Betrieb kann auch ein Schlaufenreaktor verwendet werden, in den parallel Phosgen, Sulfonsäure mit Sulfonierungsmittel und Katalysator aus der Reihe der N,N-Dialkylcarbonsäureamide eingegeben werden, während das Produkt an einem Auslaß nahe der Einlaßstelle entnommen wird.

Eine andere Variante kontinuierlicher Verfahrensführung des erfindungsgemäßen Verfahrens kann bei Verwendung einer Blasensäule durchgeführt werden. Man dosiert dann zweckmäßig die Sulfonsäure mit dem Sulfonierungsmittel am Kopf der Säule ein und führt diesem Gemisch einen Phosgenstrom von unten entgegen, während das Produkt im unteren Bereich der Säule entnommen wird.

Das im Abgas enthaltene Phosgen wird in an sich bekannter Weise, beispielsweise durch Waschen mit Dichlorbenzol, zurückgewonnen. Es kann aber auch vorteilhaft sein, die Abgaswäsche zusätzlich oder ausschließlich mit Sulfonsäure, wie Benzolsulfonsäure und/oder Sulfochlorid, wie Benzolsulfochlorid, vorzunehmen und das Phosgen beispielsweise zusammen mit der Benzolsulfonsäure, zurückzuführen.

Die Aufarbeitung des Rohprodukts erfolgt vorzugsweise durch Vakuumdestillation. Leichter flüchtige Komponenten werden vorher im schwachen Vakuum oder durch Einleiten eines Inertgases, wie z.B. Stickstoff entfernt. Überschüssiges Phosgen und gegebenenfalls der Katalysator, der ebenfalls destilliert werden kann, können zurückgeführt werden.

Eine bevorzugte Ausführungsform des erfindungsgemäßen Verfahrens besteht darin, daß man die gesamte Menge des als Katalysator beispielsweise verwendeten N,N-Dialkylcarbonsäureamids mit etwa der gleichen Volumenmenge Phosgen bei Raumtemperatur umsetzt und anschließend bei 50 bis 70°C verflüssigtes Phosgen und die Mischung aus der Sulfonsäure und dem Sulfonierungsmittel parallel so eindosiert, daß ein Phosgenüberschuß vorhanden ist.

Der als Nebenprodukt anfallende Chlorwasserstoff wird abgeleitet und kann gegebenenfalls isoliert werden. Nach Beendigung der Reaktion, die am Aufhören der Gasentwicklung festgestellt werden kann, wird überschüssiges Phosgen abgetrennt, katalytisch zersetzt oder in an sich bekannter Weise zurückgewonnen. Das verbleibende Reaktionsgemisch wird vorteilhaft durch fraktionierte Destillation unter vermindertem Druck, vorzugsweise im Bereich von 0,1—10 mbar aufgearbeitet. Soweit die eingesetzte Sulfonsäure hochsiedende Verunreinigungen, wie z.B. Diphenylsulfon, enthält, können diese aus dem Destillationsrückstand isoliert werden.

Es ist überraschend, daß bei der Umsetzung von Benzolsulfonsäure mit Phosgen in Gegenwart von Sulfonierungsmittel im praktisch quantitativer Ausbeute Sulfochloride entstehen. Gegenüber der bekannten Herstellungsweise für aromatische Sulfochloride (DT—OS 1 963 383) hat das erfindungsgemäße Verfahren den Vorteil, daß das Produkt in höherer Reinheit und in besserer Ausbeute anfällt. Außerdem wird bei der Umsetzung in Gegenwart eines Sulfonierungsmittels eine geringere Menge des Katalysators benötigt.

Die im Abgas des Verfahrens enthaltenen Nebenprodukte, Kohlendioxid und Chlorwasserstoff können leicht von mitgerissenem Phosgen, beispielsweise durch dessen katalytische Zersetzung, durch Abtrennung über Kühlfallen oder z.B. durch Extraktion mit Lösungsmitteln, wie z.B. Benzol, Toluol, Chlorbenzol oder Dichlorbenzol, befreit werden.

Durch die leichte Aufarbeitung des Abgases, ist eine geringe Belastung der Umwelt bedingt.

Beispiel 1 (Vergleichsbeispiel)

10 g Dimethylformamid werden im Kolben vorgelegt, ca. 200 g (140 ml, 2 Mol) Phosgen im kühlbaren Tropftrichter kondensiert und davon ca. 15 ml langsam zum vorgelegten Dimethylformamid eingetropft. Bei der nun ablaufenden exothermen Reaktion steigt die Temperatur im Kolben auf ca. 40°C an und es bildet sich ein weißer Kristallbrei. Man erwärmt den Kolben auf etwa 50—70°C und tropft bei dieser Innentemperatur 158 g (1,0 Mol) geschmolzene Benzolsulfonsäure (98,9%ig) innerhalb von 4 Stunden ein. Gleichzeitig wird Phosgen so eindosiert, daß es — erkennbar am starken Rückfluß — immer im Überschuß gegenüber der Sulfonsäure in der Mischung vorhanden ist.

Nach Ende des Zutropfens wird noch etwa eine Stunde lang — bis zum Ende der Gasentwicklung — bei etwa 50—70°C gerührt und bei abgestellter Kühlung über Nacht stehen gelassen.

Phosgenreste und andere flüchtige Bestandteile in der Reaktionsmischung werden bei 60°C im Wasserstrahlvakuum abgezogen und der Rückstand wird im Ölpumpenvakuum über eine verspiegelte Kolonne (3 cm × 30 cm) fraktioniert destilliert. Man erhält

10,9 g Vorlauf, Sdp. 95—97°C/0.92 mbar,

141,6 g Hauptlauf, Sdp. 97—98°C/0.66 mbar und

9,2 g Nachlauf, Sdp. 98—105°C/0,66—1,32 mbar.

Vorlauf und Hauptlauf enthalten jeweils 99,9%, der Nachlauf 96,5% Benzolsulfochlorid. Die Gesamtausbeute beläuft sich auf 91,2% der Theorie, bezogen auf eingesetzte Benzosulfonsäure (100%ig).

### Beispiele 2 bis 8

Die Beispiele 2 bis 8 wurden bei gleicher Ansatzgröße analog Beispiel 1 durchgeführt, jedoch wurde der Benzolsulfonsäure jeweils vor dem Einsatz eine definierte Menge Schwefelsäure beigemischt. Die wesentlichen Daten sind der nachstehenden Tabelle I zu entnehmen. Die Prozentangabe nach der Schwefelsäuremenge bezieht sich auf die Masse der eingesetzten Benzosulfonsäure und die Prozentwerte bei den Produktfraktionen geben den Gehalt an Benzosulfochlorid (nach GC) an.

Tabelle I

| Beispiel | DMF—Menge | $H_2SO_4$—Menge | Produktfraktionen | Gesamtausbeute |
|---|---|---|---|---|
| 2 | 10 g | 6,3 g (4%) | I ...127,3 g (99,9%ig)<br>II ....41,5 g (95,5%ig) | 95,5% |
| 3 | 10 g | 9,5 g (6%) | I ...168,5 g (99,9%ig)<br>II .....5,1 g (79,6%ig) | 97,6% |
| 4 | 10 g | 11,1 g (7%) | I ...170,8 g (99,9%ig)<br>II .....6,1 g (55,2%ig) | 98,5% |
| 5 | 10 g | 12,6 g (8%) | I ....18,7 g (98,9%ig)<br>II ...148,2 g (99,9%ig)<br>III .....9,9 g (58,1%ig) | 97,6% |
| 6 | 12 g | 15,8 g (10%) | 168,0 g (99,8%ig) | 94,9% |
| 7 | 15 g | 19,0 g (12%) | I ...164,2 g (99,5%ig)<br>II ....13,3 g (50,2%ig) | 96,3% |
| 8 | 18 g | 23,7 g (15%) | I ...168,0 g (99,8%ig)<br>II ....15,6 g (24,5%ig) | 97,1% |

### Beispiel 9

Beispiel 9 wurde analog Beispiel 1 durchgeführt mit der Abweichung, daß dem Dimethylformamid-Katalysator vor der Zugabe von Phosgen und Benzolsulfonsäure 11,1 g (7%) Schwefelsäure beigemischt wurden.

Bei der Destillation erhielt man

168,0 g Hauptlauf, Sdp. 100—102°C/0,66 mbar

10,1 g Nachlauf, Sdp. 102—113°C/6,6 mbar

Der Hauptlauf enthält 99,8%, der Nachlauf 54,3% Benzolsulfochlorid. Die Gesamtausbeute beläuft sich damit auf 98,0% der Theorie, bezogen auf eingesetzte Benzolsulfonsäure (100%ig).

Beispiele 10 bis 13

Die Beispiele 10 bis 13 wurden bei gleicher Ansatzgröße analog Beispiel 1 durchgeführt mit der Abweichung, daß der Benzolsulfonsäure jeweils vor dem Einsatz eine definierte Menge eines Sulfonierungsmittels (s. Tabelle II) beigemischt wurde.

Tabelle II

| Beispiel | Sulfonierungsmittel | Produktfraktionen | Gesamtausbeute |
|---|---|---|---|
| 10 | Schwefeltrioxid<br>9,5 g (6%) | I . . . . . . 157,9 g (99,5%ig)<br>II . . . . . . . 10,1 g (78,3%ig) | 93,5% |
| 11 | Oleum 65%ig<br>11,1 g (7%) | I . . . . . . 143,9 g (98,8%ig)<br>II . . . . . . . 26,2 g (91,2%ig) | 94,0% |

Fortsetzung

| Beispiel | Sulfonierungsmittel | Produktfraktionen | Gesamtausbeute |
|---|---|---|---|
| 12 | Chlorsulfonsäure<br>12,6 g (8%) | I . . . . . . 169,3 g (99,8%ig)<br>II . . . . . . . . 6,3 g (68,8%ig) | 98,2% |
| 13 | Sulfurylchlorid<br>15,8 g (10%) | 177,8 g (97,5%ig) | 98,2% |

Beispiele 14 bis 16

Die Beispiele 14 bis 16 werden analog Beispiel 4 durchgeführt mit der Abweichung, daß der Dimethylformamid-Katalysator in einem Lösungsmittel vorgelegt wurde und die Reaktion in diesem Lösungsmittel (siehe Tabelle III) bei einer dessen Siedepunkt angepaßten Temperatur durchgeführt wird.

Tabelle III

| Beispiel | Lösungsmittel | Temperatur | Produktfraktionen | Gesamtausbeute |
|---|---|---|---|---|
| 14 | Dichlormethan<br>150 ml | 35—45°C | I . . . . . . 170,3 g (99,6%ig)<br>II . . . . . . . . 6,6 g (73,5%ig) | 98,7% |
| 15 | Trichlormethan<br>150 ml | 50—60°C | I . . . . . . 169,2 g (99,4%ig)<br>II . . . . . . . . 7,5 g (79,9%ig) | 98,6% |
| 16 | 1,2-Dichloräthan<br>150 ml | 70—80° | I . . . . . . 162,0 g (99,7%ig)<br>II . . . . . . . . 9,1 g (79,1%ig) | 95,5% |

Beispiel 17

In einer Blasensäule (senkrecht stehendes Reaktionsrohr, 60 cm hoch, unten 6 cm und im oberen Drittel 10 cm weit, Gaseinleitungsrohr mit Fritte in 4 cm Höhe, Innenthermometer) werden 624 g (8 Mol) Benzol und 8 g Phosphorsäure vorgelegt. Die Mischung wird auf 40°C erwärmt und in der Folge zur Vermeidung von Siedeverzügen gerührt.

An einem auf die Blasensäule aufgesetzten Kühler wird über zwei Kühlfallen eine Vakuumpumpe angeschlossen und der Innendruck der Apparatur auf 300 mbar eingestellt.

320 g (4 Mol) flüssiges Schwefeltrioxid werden innerhalb von etwa 3 Stunden in einen auf 80°C erwärmten Kolben getropft; dabei verdampft das Schwefeltrioxid und wird durch die Fritte in das Benzol geleitet, wo sofort die exotherme Reaktion bei 40—50°C unter leichtem Rückfluß abläuft. Nach Beendigung des Einleitens von Schwefeltrioxid wird ein Teil der Mischung zur Analyse abgetrennt und destillativ vom Benzol befreit.

| | |
|---|---|
| Analyse: | 19,7% Gehalt an organisch gebundenem Schwefel |
| | 4,8% Diphenylsulfon |
| | 1,5% Schwefelsäure |
| | 0,7% Wasser |

Daraus errechnet sich eine Ausbeute von 93% d.Th. an Benzolsulfonsäure.

Zur Durchführung der zweiten Reaktionsstufe werden 5 g Dimethylformamid in einem Reaktionskolben vorgelegt und ungefähr 10 g kondensiertes Phosgen eingetropft. Bei einer Innentemperatur von 50—70°C werden gleichzeitig 25% der oben erhaltenen Sulfonierungsmischung (0,93 Mol Benzolsulfonsäure in 1 Mol Benzol) und ungefähr 140 g (1,4 Mol) Phosgen innerhalb von 4 Stunden eingetropft. Die Mischung wird noch ungefähr eine Stunde — bis zum Ende der Gasentwicklung — bei

60°C gerührt und bei abgestellter Kühlung über Nacht stehen gelassen. Die Aufarbeitung erfolgt analog Beispiel 1 destillativ.

Man erhält

33 g 1. Fraktion; Sdp. 30—40°C/250 mbar (Benzol 98%ig)

0,7 g 2. Fraktion; Sdp. 32—90°C/2,63 mbar

135,3 g 3. Fraktion; Sdp. 91—86°C/2,24—2,63 mbar

15,7 g 4. Fraktion; Sdp. 96°C/3,29 mbar

Die 2. Fraktion enthält 99,2%, die 3. Fraktion 99,9% und die 4. Fraktion 99,7% Benzolsulfochlorid.

Die Phosgenierungsausbeute beläuft sich damit auf 92,2%, die Gesamtausbeute über zwei Stufen auf 86% der Theorie, bezogen auf eingesetztes Schwefeltrioxid.

Beispiel 18

In einem Reaktionskolben mit Innenthermometer, Begasungsrührer und Rückflußkühler werden 624 g (8 Mol) Benzol und 8 g Phosphorsäure vorgelegt. An den Kühler wird über zwei Kühlfallen eine Vakuumpumpe angeschlossen und der Innendruck der Apparatur auf 300 mbar eingestellt. 320 g (4 Mol) flüssiges Schwefeltrioxid werden innerhalb von drei Stunden in einen auf 80°C erwärmten Kolben getropft. Dabei verdampft das Schwefeltrioxid und wird über den Begasungsrührer bei einer Innentemperatur zwischen 40 und 50°C in das Benzol geleitet. Nach Beendigung des Einleitens von Schwefeltrioxid wird das nicht umgesetzte Benzol zunächst bei Normaldruck, dann im Vakuum abdestilliert.

Analyse des Sulfonierungsprodukt es:

18,7 g S org. (theor. 20,3%)
8,9 g Diphenylsulfon
3,9% Schwefelsäure
0,3% Wasser

Damit ergibt sich eine Sulfonierungsausbeute von 87% der Theorie an Benzolsulfonsäure.

Die Umsetzung des Sulfonierungsproduktes mit Phosgen wird analog Beispiel 17, jedoch bei einer Innentemperatur zwischen 20 und 30°C durchgeführt. Vor der Phosgenierung werden zum Sulfonierungsprodukt 12 g Schwefelsäure gegeben. Es werden 22 g Dimethylformamid-Katalysator eingesetzt.

Nach destillativer Aufarbeitung erhält man

215,5 g 1. Fraktion; Sdp. 109—111°C/1,97 mbar

15,0 g 2. Fraktion; Sdp. 111—115°C/5,26 mbar

Die Fraktion enthält 99,1%, die zweite Fraktion 75,0% Benzolsulfochlorid.

Die Phosgenierungsausbeute beläuft sich damit auf 97,5% bei einem Ansatz von 1,30 Mol in der zweiten Stufe; die Gesamt-ausbeute über zwei Stufen beträgt 85% der Theorie, bezogen auf eingesetztes Schwefeltrioxid.

**Patentansprüche:**

1. Verfahren zur Herstellung von Sulfonsäurechloriden der Formel

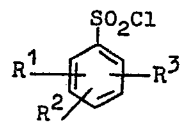

(I)

worin

$R^1$, $R^2$ und $R^3$ gleich oder verschieden sind und Wasserstoff, einen niederen Alkyl- oder einen Cycloalkylrest, Halogen, Nitro, Aryl, Aralkyl, Arylether oder einen Rest

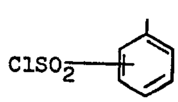 , 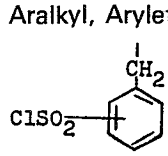 oder 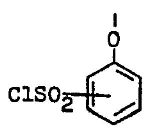 bedeuten,

wobei die Reste $R^1$, $R^2$ und $R^3$ durch Halogen, Nitro, Niederalkyl, Aryl, Aroxy, Alkoxy oder Aralkyl substituiert sein können,
oder wo benachbarte Reste
$R^1$ und $R^2$ zu einem gegebenenfalls durch eine Sulfonsäurechloridgruppe substituierten cycloaliphatischen oder aromatischen carbocyclischen Ring verknüpft sind
aus Sulfonsäure der Formel

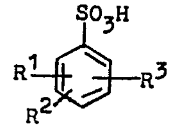 (II)

worin
$R^1$, $R^2$ und $R^3$ die oben genannte Bedeutung haben und Phosgen in Gegenwart von Katalysatoren aus der Klasse der N,N-Dialkylcarbonsäureamide, dadurch gekennzeichnet, daß man die Umsetzung in Gegenwart eines Sulfonierungsmittels durchführt.

2. Verfahren nach Anspruch 1, dadurch gekennzeichnet, daß man als Sulfonierungsmittel Schwefelsäure, Schwefeltrioxid, Chlorsulfonsäure, Sulfurylchlorid oder Gemische dieser Verbindungen verwendet.

3. Verfahren nach Ansprüchen 1 und 2, dadurch gekennzeichnet, daß man bis zu etwa 15% Sulfonierungsmittel, bezogen auf die eingesetzte Sulfonsäure, verwendet.

4. Verfahren nach Ansprüchen 1 bis 3, dadurch gekennzeichnet, daß man die Reaktion bei Temperaturen bis 100°C durchführt.

## Revendications

1. Procédé de préparation de chlorures d'acides sulfoniques de formule

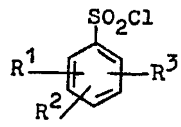 (I)

dans laquelle
$R^1$, $R^2$ et $R^3$, identiques ou différents, représentent l'hydrogène, un groupe alkyle inférieur ou cycloalkyle, un halogène, un groupe nitro, aryle, aralkyle, aryléther ou un reste

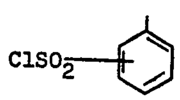 , 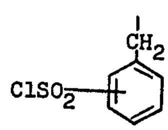 ou 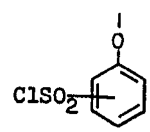

les restes $R^1$, $R^2$ et $R^3$ pouvant porter des substituants halogéno, nitro, alkyle inférieur, aryle, aroxy, alcoxy ou aralkyle,
ou bien des restes
$R^1$ et $R^2$ voisins pouvant être reliés avec formation d'un noyau cycloaliphatique ou aromatique homogène éventuellement substitué par un groupe chlorure d'acide sulfonique,
à partir d'un acide sulfonique de formule

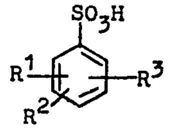 (II)

dans laquelle
$R^1$, $R^2$ et $R^3$ ont les significations indiquées ci-dessus, et de phosgène, en présente de catalyseurs pris dans la classe des N,N-dialkylcarboxamides, ce procédé se caractérisant en ce que la réaction est effectuée en présence d'un agent sulfonant.

2. Procédé selon la revendication 1, caractérisé en ce que l'on utilise en tant qu'agent sulfonant l'acide sulfurique, l'anhydride sulfurique, l'acide chlorosulfonique, le chlorure de sulfuryle ou un mélange de ces composés.

3. Procédé selon les revendications 1 et 2, caractérisé en ce que l'on utilise jusqu'à 15% environ d'agent sulfonant par rapport à l'acide sulfonique mis en oeuvre.

4. Procédé selon les revendications 1 à 3, caractérisé en ce que l'on effectue la réaction à des températures allant jusqu'à 100°C.

**Claims**

1. Process for the preparation of sulphonic acid chlorides of the formula

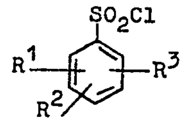

(I)

wherein

$R^1$, $R^2$ and $R^3$ are identical or different and denote hydrogen, a lower alkyl radical or a cycloalkyl radical, halogen, nitro, aryl, aralkyl, aryl ether or a radical

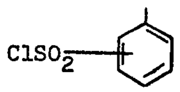 , 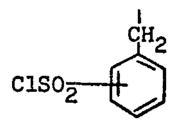 or 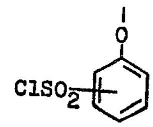

the radicals $R^1$, $R^2$ and $R^3$ being able to be substituted by halogen, nitro, lower alkyl, aryl, aroxy, alkoxy or aralkyl,

or wherein

adjacent radicals $R^1$ and $R^2$ are linked to form a cycloaliphatic or aromatic carbocyclic ring which is optionally substituted by a sulphonic acid chloride group,

from sulphonic acids of the formula

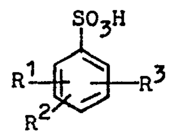

(II)

wherein

$R^1$, $R^2$ and $R^3$ have the abovementioned meaning, and phosgene in the presence of catalysts of the N,N-dialkylcarboxylic acid amide class, characterised in that the reaction is carried out in the presence of a sulphonating agent.

2. Process according to Claim 1, characterised in that sulphuric acid, sulphur trioxide, chlorosulphonic acid, sulphuryl chloride or mixtures of these compounds are used as the sulphonating agents.

3. Process according to Claims 1 and 2, characterised in that up to about 15% of sulphonating agent, relative to the sulphonic acid employed, is used.

4. Process according to Claims 1 to 3, characterised in that the reaction is carried out at temperatures up to 100°C.